# EUROPÄISCHE PATENTSCHRIFT

(11) **EP 1 103 241 B1**
(45) Veröffentlichungstag und Bekanntmachung des Hinweises auf die Patenterteilung: **16.07.2008**
(21) Anmeldenummer: 00125106.5
(22) Anmeldetag: 17.11.2000
(51) Int. Cl.: A61F 15/00, A61F 17/00

(54) **Verpackung für Pflaster**
Package for dressings
Emballage pour pansements

(30) Priorität: 26.11.1999 DE 19956917
(43) Veröffentlichungstag der Anmeldung: 30.05.2001
(73) Patentinhaber: Lohmann & Rauscher GmbH & Co. KG, 56567 Neuwied (DE)
(72) Erfinder: Anhäuser, Dieter, 56581 Melsbach (DE); Kreucher, Peter, 53498 Bad Breisig (DE); Lauth, Andreas, 56579 Rengsdorf (DE); Thewalt, Joachim, 56581 Melsbach (DE)
(74) Vertreter: Flaccus, Rolf-Dieter

(56) Entgegenhaltungen:
- EP-A- 0 418 607
- EP-A- 0 922 651
- DE-A- 19 828 178
- DE-U- 29 606 678
- US-A- 4 574 951
- US-A- 4 607 633
- US-A- 4 666 040
- US-A- 5 091 035
- US-A- 5 133 477

## Beschreibung

Die Erfindung betrifft eine Verpackung für Pflaster, umfassend einen Träger aus vergleichsweise steifem, dünnem Material und reversiblem Verschluß.

Verpackungen für eine nicht festzulegende Anzahl einzelner Pflaster sind Stand der Technik. Beispielsweise beschreibt US 5,133,477 ein Verpackungs- und Spendersystem für eine Vielzahl von Fingerbandagen, umfassend ein Trägermaterial als Rückschicht mit einer haftklebenden Beschichtung und darauf angehefteten Wundpflastern, weiterhin eine Ablöseschicht auf einer exponierten, adhäsiven Oberfläche und dem Pflaster, deren andere exponierte adhäsive Oberfläche lösbar an dem als Ablöseunterlage ausgerüsteten Basismaterial befestigt ist, und einen die Wundpflaster überdeckenden Deckel, der schwenkbar am Basisträger angelenkt ist und aus durchsichtigem Kunststoff besteht.

DE 44 40 727 A1 beschreibt eine Pflasterverpackung für Einzelpflaster aus einem Trägermaterial, welches einseitig mit einer Selbstklebeschicht versehen ist und gegebenenfalls mittig eine Wundauflage trägt, mit einer unteren und einer oberen Decklage, welche allseits über die Pflaster hinausragen und in ihren Randbereichen trennbar miteinander versiegelt sind, die dadurch gekennzeichnet ist, daß die Verpackung mindestens zwei übereinander angeordnete Pflaster enthält, sich zwischen den einzelnen Pflastern jeweils eine Zwischenlage befindet aus einem Trägermaterial, das zumindest in seinen Randbereichen siegelbar beziehungsweise schweißbar ist, und diese Zwischenlagen mit den Decklagen an ihren Randbereichen allseitig durch eine gemeinsame Siegelnaht verbunden sind, wobei die Lagen einzeln wieder trennbar sind. Vorzugsweise haften die Pflaster zumindest in einem Teilbereich auf der Oberseite der unteren Decklage beziehungsweise der jeweiligen Zwischenlage.

WO 95/180 46 sieht eine ummantelte, mehrfach gefaltete Trägerkarte mit darauf angeordneten sterilen Artikeln wie adhäsive Verbandstreifen vor.

US 5,161,687 beschreibt eine verkaufsorientierte Verpackung für blockgestanzte Aufklebezettel mit einer steifen Karte als Rückschicht sowie mit einer Aufhängeöse und darauf angehefteten Aufklebezetteln sowie mit einer transparenten, mehrfach lösbaren und wiederbefestigbaren Deckschicht aus durchsichtigem Material. Die Packung kann auch vielschichtig sein.

Ausgehend vom vorgenannten Stand der Technik liegt der Erfindung die Aufgabe zugrunde, eine Verpackung für Pflaster anzugeben, die wesentlich einfacher in der Ausbildung und im wesentlichen nur aus einem einteiligem Träger herstellbar ist, die geeignet zur beliebig oft erfolgenden Entnahme von Pflastern ist, und die in Größe und Aufmachung geeignet ist, in einem Portemonnaie oder einer Tasche einer Person zum ständig zugreifbaren Gebrauch tragbar beziehungsweise unterbringbar zu sein.

Zur Lösung der Aufgabe wird mit der Erfindung eine Ausgestaltung der Verpackung für Pflaster vorgeschlagen, welche die Merkmale der Erfindung entsprechend dem Kennzeichnungsteil von Anspruch 1 aufweist.
Unter einem Heftchen wird eine durch Umfalten entstandene, scharnierartige Verbindung, die mindestens zwei Trägerteilabschnitte, die mittels eines Verschlusses zusammengehalten werden, verstanden, wobei innerhalb des Heftchens Pflasterabschnitte bevorratet werden.

Im Vergleich zum Stand der Technik ist die Verpackung für Pflaster nach der Erfindung von äußerst überraschender, unübertreffbarer Einfachheit. De facto besteht sie aus einem Träger, dessen Formgebung zu einem die Pflaster umschließenden Heftchen mit einem reversiblen Verschluß besteht.

Darüber hinaus bietet die Verpackung nach der Erfindung den signifikanten Vorteil, daß das gefaltete und mit Pflastern bestückte geschlossene Heftchen vorzugsweise eine Größe besitzt, die eine Aufbewahrung ähnlich derjenigen z.B. einer Scheckkarte im Portemonnaie oder in einer Brieftasche oder Anzugtasche einer Person ermöglicht.
Dabei können die Konturen des Heftchens jede zweckdienliche Form wie z. B. rund, dreieckig, viereckig annehmen.
Ebenso beinhaltet die Erfindung die Ausgestaltung einer Mehrfachfaltung wie z. B. die Leporello-Faltung.

Der Verschluß des Heftchens kann beispielsweise ein Klettverschluß, ein Druckknopfverschluß oder ein Haftklebverschluß sein. Er kann durch Anbringen eines Haftklebauftrags beispielsweise randseitig oder mittig und/oder musterförmig auf die Innenseite des Heftchens ausgebildet sein.

Eine bevorzugte Ausführungsform ist, daß sich der Verschluß 13 des Heftchens 9 auf der den Pflastern abgewandten Seite des Trägers 1 befindet. Durch zweimaliges Umfalten des Trägers entsteht ein über eine Seite des Heftchens überstehendes Stück 10, das an einem Haftklebauftrag 12 auf der Außenseite des zuerst umgefalteten Trägerteils 1', unter Ausbildung eines reversiblen Verschlusses 13 anheftbar ausgebildet ist. Die Fläche des Haftklebauftrags 12 ist in einer bevorzugten Ausführungsform kleiner als die Fläche des Verschlußstreifens 11 und bildet damit eine Anfaßhilfe zum Öffnen des Heftchens 9 aus.

In einer weiteren Ausführungsform kann, wie aus DE 37 11 256 A1, DE 39 31 019 A1 oder DE 39 31 018 A1 bekannt ist, der Träger 1 mit Sollbruchlinien versehen sein, die Entnahmehilfen für die Pflaster 2 darstellen.

Entsprechend Ausgestaltungen der Erfindung kann das Material des Trägers beispielsweise aus Kunststoff oder Papier oder Metall oder einer Kombination daraus bestehen. Dabei ist es dem Fachmann anheim gestellt, weitere geeignete Materialien als Träger für die Einzelpflaster auszuwählen und vorzusehen.
Der Träger kann dabei eine Dicke zwischen 5 und 5000 µm besitzen, bevorzugt eine Dicke zwischen 100 und 250 µm.
Eine weitere Ausgestaltung besteht darin, daß der Träger 1 transparent und/oder durchsichtig und/oder farbig ist.
Eine Ausgestaltung sieht vor, daß die Innenseite des faltbaren Heftchens mit einer Silikonbeschichtung von ca. 5 µm Dicke versehen ist.

Schließlich kann von der Maßnahme Gebrauch gemacht sein, daß die erfindungsgemäße Verpackung an den Außenseiten beispielsweise eine Beschriftung trägt, die nützliche Angaben zur Person wie Blutgruppe oder Hinweise auf Krankheiten (z.B. Zuckerkrankheit beziehungsweise Herzinsuffizienz etc.), oder eine Kurzanleitung zum Blutstillen etc. zum Gegenstand hat.

Weitere Einzelheiten, Merkmale und Vorteile der Erfindung ergeben sich aus der nachstehenden Erläuterung eines in den Zeichnungen schematisch dargestellten Ausführungsbeispieles. Es zeigen:
Figur 1 eine Ausführungsform in perspektivischer Ansicht einer Verpackung in geöffnetem Zustand;
Figur 2 die Verpackung gemäß Fig. 1 in Draufsicht von oben;
Figur 3 eine alternative Ausführung der Verpackung mit einem randständigen Dichtungsband im geschlossenen Zustand der Verpackung.

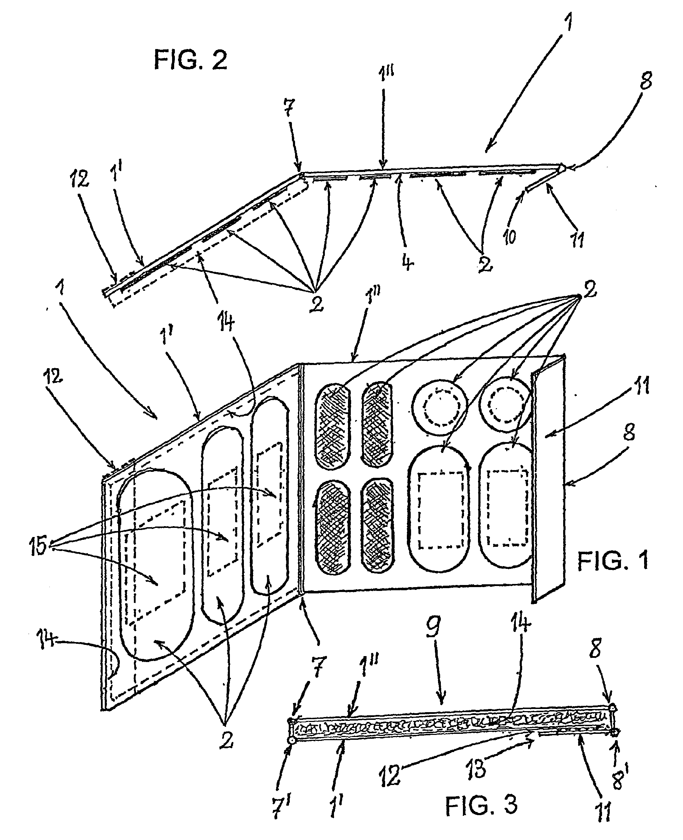

Die in der Figur 1 dargestellte Verpackung für Pflaster 2 umfaßt einen Träger 1 aus vergleichsweise steifem, dünnem Material.
Erfindungsgemäß ist der Träger 1 an einer Seite 4, wie in Fig. 2 gezeigt, mit einer Silikonbeschichtung augerüstet und die Pflaster 2 sind mit einer haftklebenden Beschichtung auf jeweils einem halben Flächenbereich 1', 1" dieser Seite ablösbar angeheftet.
Dabei sind die halben Flächenbereiche 1', 1" des Trägers 1 in einer ersten Falzlinie 7 um 180°C unter Ausbildung eines die Pflaster 2 umschließenden Heftchens 9 gegeneinander gefaltet, wie dies beispielsweise der Figur 3 zu entnehmen ist.
Gegenüberliegend der ersten Falzlinie 7 ist ein über die Breitseite des Heftchens 9 überstehendes Stück Trägermatterial 10, wie aus der Figur 2 entnehmbar, in einer zweiten Falzlinie 8 unter Ausbildung eines Verschlußstreifens 11 um 180 °C umfaltbar und an einem Haftklebauftrag 12 auf der Außenseite des zuerst umgefalteten Trägerblattes 1' unter Ausbildung eines reversiblen Verschlusses 13 anheftbar ausgebildet.

Eine besonders vorteilhafte Ausgestaltung der Verpackung ist, daß das gefaltete und mit Pflastern 2 bestückte, geschlossene Heftchen 9 eine Größe besitzt, die eine Aufbewahrung ähnlich derjenigen einer Scheckkarte im Portemonnaie oder in einer Brieftasche oder Anzugstasche einer Person ermöglicht.

Wenn die Verpackung mit darauf angeordneten Pflasterstrips 2 und darauf beispielsweise Auflagen wie blutstillende Beschichtungen etc. besitzt, die im Anwendungsfall zu Abdekkung einer kleinen Wunde oder einer Scheuerstelle beziehungsweise einer Aufschürfung verwendet werden, dann kann vermieden werden, daß die Pflaster beziehungsweise die darauf befindlichen Auflagen 15 mit Staub oder Bakterien oder sonstigen ungünstigen Umwelteinflüssen kontaminiert werden. Zu diesem Zweck muß verhindert werden, daß in das geschlossene Heftchen 9 Schadstoffe oder Schmutzteilchen eindringen können.
Um dies zu vermeiden, kann von der Maßnahme Gebrauch gemacht werden, daß der Träger 1 etwa die Steifigkeit einer Kreditkarte aufweist, dessen einer Flächenbereich (zum Beispiel 1') randständig von einem Dichtungsband 14 beispielsweise in Form eines flachen Vliesstoff-Steges umgeben ist, wobei dann die gelenkige Verbindung 7, 8 zwischen den Flächenbereichen 1', 1" im Abstand gemäß der Höhe des Dichtstreifens 14, wie dies in der Figur 3 dargestellt ist, als Doppelfalze 7, 7'; 8, 8' ausgebildet ist, derart, daß die zum Heftchen 9 zusammengefaltete Verpackung ringsum gegen das Eindringen äußerer Einflüsse der vorgängig genannten Art abgeschlossen ist.
Vorzugsweise kann dabei der Dichtungsstreifen 14 bakterizid ausgerüstet sein.

Die Verpackung nach der Erfindung ist sehr einfach und zweckmäßig und löst in optimaler Weise die eingangs gestellte Aufgabe.

## Patentansprüche

1. Verpackung für Pflaster, umfassend einen Träger (1) mit darauf aufgebrachten Einzelpflastern, wobei der Träger (1) durch zweimaliges Umfalten über Falzlinien (7), (8) in zwei Flächenbereiche (1'), (1 ") und einen einzelnen Verschlußstreifen (11) derart gefaltet ist, daß die aufgebrachten Einzelpflaster (2) unter Ausbildung eines die Pflaster (2) umschließenden Heftchens, das einen Klett- oder Druckknopfverschluß aufweist oder bei dem der Flächenbereich (1') einen Haftklebeauftrag (12) auf der den Pflastern abgewandten Seite des Trägers (1) oder der Verschlußstreifen (11) einen Haftklebeauftrag auf der Innenseite des Heftchens aufweist, reversibel abgedeckt sind.

2. Verpackung nach Anspruch 1, **dadurch gekennzeichnet, daß** die Fläche des Haftklebeauftrags (12) kleiner ist als die Fläche des Verschlußstreifens (11) und somit eine Anfaßhilfe zum Öffnen des Heftchens (9) ausbildet.

3. Verpackung nach Anspruch 1 oder 2, **dadurch gekennzeichnet, daß** das Material des Trägers (1) aus Kunststoff, Papier, Metall oder aus einer Kombination daraus besteht.

4. Verpackung nach einem oder mehreren der Ansprüche 1 bis 3, **dadurch gekennzeichnet, daß** der Träger (1) transparent und/oder durchsichtig und/oder farbig ist.

5. Verpackung nach einem oder mehreren der Ansprüche 1 bis 4, **dadurch gekennzeichnet, daß** der Träger (1) eine Dicke zwischen 5 und 5000 µm besitzt.

6. Verpackung nach Anspruch 5, **dadurch gekennzeichnet, daß** der Träger (1) eine Dicke zwischen 100 und 250 µm besitzt.

7. Verpackung nach einem oder mehreren der Ansprüche 1 bis 6, **dadurch gekennzeichnet, daß** die Innenseite des faltbaren Heftchens (9) mit einer Silikonbeschichtung von ca. 5 µm Dicke versehen ist.

8. Verpackung nach einem oder mehreren der Ansprüche 1 bis 7, **dadurch gekennzeichnet, daß** das mit Pflastern (2) versehene, verschlossene Heftchen (9) die Größe einer Scheckkarte besitzt.

9. Verpackung nach einem oder mehreren der Ansprüche 1 bis 8, **dadurch gekennzeichnet, daß** die Außenseiten des Heftchens (9) zum Anbringen nützlicher Informationen dienen.

10. Verpackung nach einem oder mehreren der Ansprüche 1 bis 9, **dadurch gekennzeichnet, daß** die Kanten des Heftchens (9) zur Vermeidung von Verschmutzungen mit einem Dichtungsband (14) ausgestattet sind.

11. Verpackung nach Anspruch 10, **dadurch gekennzeichnet, daß** das Dichtungsband (14) bakterizid ausgerüstet ist.

## Claims

1. Package for plasters, comprising a carrier (1) with individual plasters applied thereto, said carrier (1) being folded into two surface regions (1'), (1") and a single closing strip (11) by folding said carrier (1) twice along fold-lines (7), (8) such that the individual plasters (2) applied thereto are reversibly covered, thus forming a booklet which encloses the plasters (2) and which is provided with a hook-and-loop fastener or a snap fastener, or the surface region (1') of which is provided with a pressure-sensitive adhesive coating (12) on the side of the carrier (1) which is averted from the plasters, or the closing strip (11) of which is provided with a pressure-sensitive adhesive coating on the inner side of the book-let.

2. Package according to claim 1, **characterised in that** the area of the pressure-sensitive adhesive coating (12) is smaller than the area of the closing strip (11) and thus forms a gripping aid for opening the booklet (9).

3. Package according to claim 1 or 2, **characterised in that** the material of the carrier (1) consists of plastic, paper, metal or of a combination thereof.

4. Package according to one or more of claims 1 to 3, **characterised in that** the carrier (1) is transparent and/or translucent and/or coloured.

5. Package according to one or more of claims 1 to 4, **characterised in that** the carrier (1) has a thickness of between 5 and 5000 µm.

6. Package according to claim 5, **characterised in that** the carrier (1) has a thickness of between 100 and 250 µm.

7. Package according to one or more of claims 1 to 6, **characterised in that** the inner side of the foldable booklet (9) is provided with a silicone coating of about 5 µm thickness.

8. Package according to one or more of claims 1 to 7, **characterised in that** the closed booklet (9), which is provided with plasters (2), has the size of a cheque card.

9. Package according to one or more of claims 1 to 8, **characterised in that** the outer sides of the booklet (9) serve to apply useful information.

10. Package according to one or more of claims 1 to 9, **characterised in that** the edges of the booklet (9) are provided with a sealing strip (14) to avoid dirtying.

11. Package according to claim 10, **characterised in that** the sealing strip (14) is provided with a bactericidal finish.

## Revendications

1. Emballage pour pansements, comprenant un support (1) avec des pansements individuels appliqués dessus, le support (1) étant plié de manière telle, par pliage double au niveau de lignes de pliage (7), (8) en deux parties de surface (1'), (1") et une bande de fermeture (11) distincte, que les pansements individuels (2) appliqués dessus sont recouverts de manière réversible avec formation d'une pochette renfermant les pansements (2), laquelle comporte une fermeture velcro ou par bouton pression ou dans laquelle la partie de surface (1') comporte une couche adhésive (12) sur le côté du support (1) détourné des pansements ou la bande de fermeture (11) comporte une couche adhésive sur le côté intérieur de la pochette.

2. Emballage selon la revendication 1, **caractérisé en ce que** la surface de la couche adhésive (12) est inférieure à la surface de la bande de fermeture (11) et constitue ainsi une aide à la préhension permettant d'ouvrir la pochette (9).

3. Emballage selon la revendication 1 ou 2, **caractérisé en ce que** la matière du support (1) se compose de matière plastique, de papier, de métal ou d'une combinaison de ces matières.

4. Emballage selon une ou plusieurs des revendications 1 à 3, **caractérisé en ce que** le support (1) est transparent et/ou translucide et/ou coloré.

5. Emballage selon une ou plusieurs des revendications 1 à 4, **caractérisé en ce que** le support (1) a une épaisseur comprise entre 5 et 5000 µm.

6. Emballage selon la revendication 5, **caractérisé en ce que** le support (1) a une épaisseur comprise entre 100 et 250 µm.

7. Emballage selon une ou plusieurs des revendications 1 à 6, **caractérisé en ce que** le côté intérieur de la pochette pliable (9) est pourvu d'un revêtement en silicone d'environ 5 µm d'épaisseur.

8. Emballage selon une ou plusieurs des revendications 1 à 7, **caractérisé en ce que** la pochette (9) fermée, garnie de pansements (2), a la taille d'une carte-chèque.

9. Emballage selon une ou plusieurs des revendications 1 à 8, **caractérisé en ce que** les côtés extérieurs de la pochette (9) servent à apposer des informations utiles.

10. Emballage selon une ou plusieurs des revendications 1 à 9, **caractérisé en ce que** les bords de la pochette (9), pour éviter des souillures, sont pourvus d'une bande d'étanchéité (14).

11. Emballage selon la revendication 10, **caractérisé en ce que** la bande d'étanchéité (14) est bactéricide.
